# EUROPEAN PATENT APPLICATION

(11) **EP 1 679 067 A1**
(43) Date of publication of application: **12.07.2006**
(21) Application number: 04773372.0
(22) Date of filing: 16.09.2004
(51) Int. Cl.: A61K 31/195, A61K 31/216, A61K 31/4365, A61K 31/519, A61K 31/616, A61K 45/00, A61P 7/02, A61P 9/04, A61P 9/10, A61P 11/00, A61P 43/00

(54) **CONCURRENT DRUGS**

(30) Priority: 19.09.2003 JP 2003328487
(71) Applicant: Kissei Pharmaceutical Co., Ltd., Matsumoto-shi Nagano 399-8710 (JP)
(72) Inventor: HOYANO, Yuji, Kissei Pharmaceutical Co., Ltd., Minamiazumi-gun, Nagano 399-8304 (JP); HONMA, Toshiki, Kissei Pharmaceutical Co., Ltd., Minamiazumi-gun, Nagano 399-8304 (JP); KOIZUMI, Takashi, Kissei Pharmaceutical Co., Ltd., Minamiazumi-gun, Nagano 399-8304 (JP); AKAHANE, Satoshi, Kissei Pharmaceutical Co., Ltd., Minamiazumi-gun, Nagano 399-8304 (JP)
(74) Representative: Hayes, Adrian Chetwynd
(86) International application number: PCT/JP2004/013982
(87) International publication number: WO 2005/027896

(57) **Abstract**

The present invention provides a combination drug which is useful for the prevention or treatment of thromboembolism. A pharmaceutical agent comprising an antiplatelet agent in combination with a 5-amidino-2-hydroxybenzenesulfonamide derivative represented by the general formula: 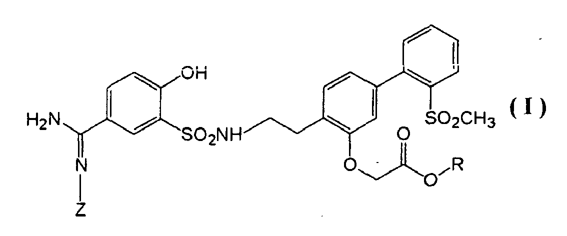 wherein R represents a hydrogen atom or a lower alkyl group, and Z represents a hydrogen atom or a hydroxy group, or a pharmaceutically acceptable salt thereof.

## Description

### Field of the Invention

The present invention relates to a pharmaceutical agent comprising an antiplatelet agent in combination with a 5-amidino-2-hydroxybenzenesulfonamide derivative or a pharmaceutically acceptable salt thereof.

More particularly, the present invention relates to a pharmaceutical agent comprising an antiplatelet agent in combination with a 5-amidino-2-hydroxybenzenesulfonamide derivative represented by the general formula: 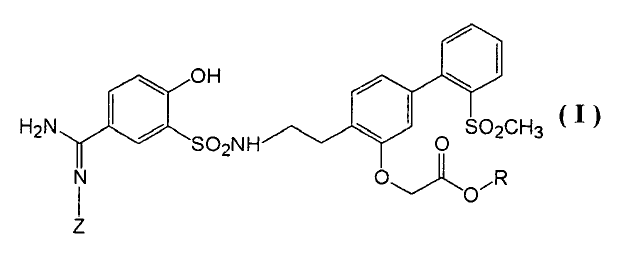 wherein R represents a hydrogen atom or a lower alkyl group; and Z represents a hydrogen atom or a hydroxy group; or a pharmaceutically acceptable salt thereof, which is a direct and selective inhibitor of activated blood coagulation factor X (hereinafter referred to as "factor Xa").

### Background Art

In recent years, it have been found that activation of blood platelet plays an important role in thrombus formation, therefore, antiplatelet agents which decreases the activation of blood platelet have been used for prevention or treatment of the various diseases in which thrombus formation participate. As the diseases in which thrombus formation participate, thromboembolism caused directly or indirectly by forming thrombus in blood vessel can be illustrated, which is classified into 1) thrombosis; diseases caused by blockade of the blood flow with formed thrombus which has been formed and gradually gotten big, for example, unstable angina, cardiac infarction, cerebral thrombosis and the like, or 2) embolism: diseases caused by clogging up the blood vessel with thrombus which has been formed and carried by the bloodstream, for example, economy class syndrome, cardiogenic embolism and the like (Non-patent literature 1). As mentioned above, since these thromboembolisms are initiated by two factor, thrombosis and embolism, it is not always possible to obtain satisfiable effect by only improving the blood flow at occlusion site, therefore, it is important to block the growing or carrying of thrombus by inhibiting its formation. At the present, aspirin, cilostazol, dipyridamole, ticlopidine, clopidogrel and the like are used as antiplatelet agents for the prevention or treatment of thromboembolism. However, since an antiplatelet agent has a risk of bleeding tendency while it inhibits the thrombus formation (Non-patent literatures 2 and 3), it is hard to increase the dosage of an antiplatelet agent for its inhibition effect of the thrombus formation. Thus, it is desired that a more improved agent for the prevention or treatment for thromboembolism.

For example, same examples of the combination of an antiplatelet agent and a factor Xa inhibitor are proposed as effective for the prevention or treatment of thromboembolism in the following literatures.
(a) An example combination of a low molecular weight heparin or a heparinoid derivative which are indirect factor Xa inhibitors and a platelet aggregation inhibitor (Patent literature 1).
(b) An example combination of a low molecular weight heparin which is an indirect factor Xa inhibitor and a glycoprotein-IIb/IIIa antagonist (Patent literature 2).
(c) An example combination of a synthetic oligosaccharide which is an indirect factor Xa inhibitor or DX-9065a which is a direct factor Xa inhibitor represented by the formula: 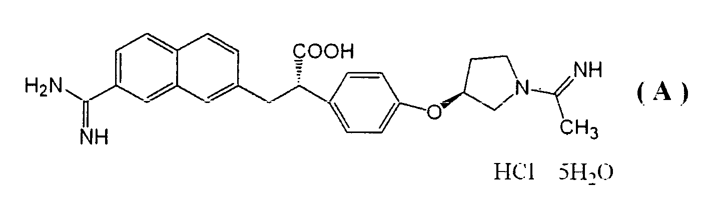 and aspirin, ticlopidine, dipyridamole, clopidogrel or cilostazol (Patent literature 3).
(d) An example combination of DX-9065a which is a direct factor Xa inhibitor represented by the above formula (A) or YM-60828 represented by the formula: 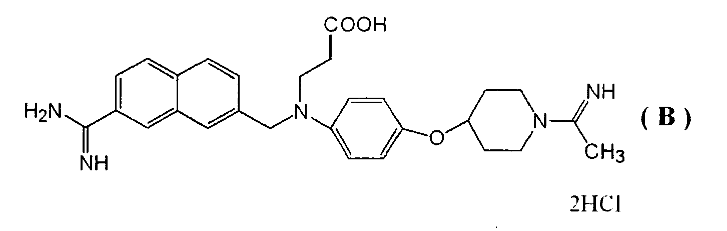 or, a combination of a compound which is a direct factor Xa inhibitor represented by the general formula: 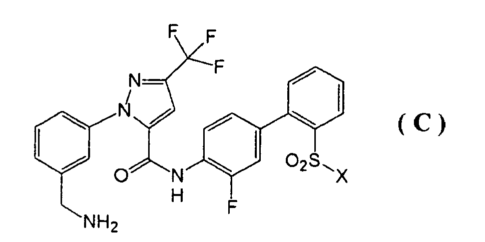 wherein X represents a methyl group or an amino group, and aspirin or glycoprotein-IIb/IIIa inhibitor (Patent literature 4).
(e) An example combination of DPC423 which is a direct factor Xa inhibitor represented by the formula: 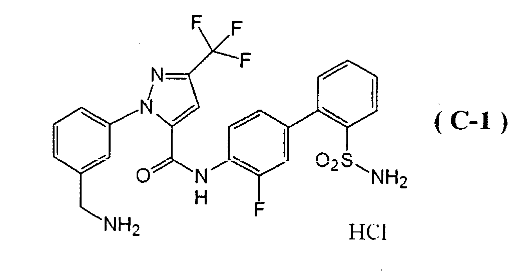 and aspirin (Non-patent literature 4).

However, the factor Xa inhibitors proposed in the above example combinations are indirect factor Xa inhibitors, which inhibition mechanism is entirely different from the direct factor Xa inhibitors of the present invention, or are agents having their entirely different chemical structures are from the structure of a 5-amidino-2-hydroxybenzenesulfonamide derivative represented by the above general formula (I) of the present invention.

In addition, the effects shown in the above example combinations is just a effect to improve the blood flow in a thrombus formation site in an arterial thrombosis model mentioned in the above combinations, and there is no report that shows 1) a decreasing effect on the amount of thrombus formation; 2) an improving effect on hypercoagulable state; which improvings are shown in the present invention.

As mentioned above, it is completely unknown that a combination of an antiplatelet agent and 5-amidino-2-hydroxybenzenesulfonamide derivative represented by the above general formula (I) is a preferable combination for the prevention or treatment of thromboembolism.

The purpose of the present invention is to provide an excellent combination drug having a prevention or treatment effect on thromboembolism.
Patent literature
1. Tokuhyo No.JP2001-506603
2. Tokuhyo No.JP2003-526617
3. Tokuhyo No.JP2002-504110
4. Tokuhyo No.JP2002-538226
Non-patent literaure
1. The Merck Manual 17th edition Japanese version, Section 11 Chapter 132, Thrombotic Disease
2. Masako Waki and another 1 person, Rinsho Kagaku, 1998, Vol.34, No.1, pp52-60
3. Yasuo Ikeda, Medicinal Practice, 2002, Vol.19, No.5, pp839-844
4. PANCRAS C. WONG and another 8 persons, Journal of Pharmacology and Experimental Therapeutics, 2002, Vol.303, No.3, pp993-1000

### Disclosure of the Invention

The present inventors studied earnestly basing aforementioned problems, as a result, have found that using an antiplatelet agent in combination with a certain kind of 5-amidino-2-hydroxybenzenesulfonamide derivative exerts two significant effects, 1) a synergistically increased effect decreasing the amount of thrombus formation and 2) a superior improving effect which is not shown by use a single application on hypercoagulable state. The present inventors have further studied based on these knowledge, which resulted in forming the present invention.

Therefore, the present invention relates to:
[1] a pharmaceutical agent comprising an antiplatelet agent in combination with a 5-amidino-2-hydroxybenzenesulfonamide derivative represented by the above general formula (I) or a pharmaceutical acceptable salt thereof;
[2] a pharmaceutical agent as described in the above [1], wherein the antiplatelet agent is a compound selected from the group consisting of aspirin, dipyridamole, cilostazol, ticlopidine and clopidogrel;
[3] a pharmaceutical agent as described in the above [1], wherein the antiplatelet agent is a compound selected from the group consisting of aspirin, dipyridamole, cilostazol and ticlopidine;
[4] a pharmaceutical agent as described in the above [1], wherein the 5-amidino-2-hydroxybenzenesulfonamide derivative is a compound selected from the group consisting of n-butyl [4-[2-[2-hydroxy-5-(N-hydroxycarbamimidoyl)-benzenesulfonylamino]ethyl]-2'-methanesulfonylbiphenyl -3-yloxy]acetate, [4-[2-[2-hydroxy-5-(N-hydroxycarbamimidoyl)benzenesulfonylamino]ethyl]-2'-methanesulfonylbiphenyl-3-yloxy]acetic acid, n-butyl [4-[2-(5-amidino-2-hydroxybenzenesulfonylamino)ethyl]-2'-methanesulfonylbiphenyl-3-yloxy]acetate and [4-[2-(5-amidino-2-hydroxybenzenesulfonylamino)ethyl]-2'-methanesulfonylbiphenyl-3-yloxy]acetic acid;
[5] a pharmaceutical agent of any one of [1] to [4] described above, which is an agent for the prevention or treatment of thromboembolism;
[6] a pharmaceutical agent as described in the above [5] , wherein the thromboembolism is arterial thromboembolism;
[7] a pharmaceutical agent as described in the above [5], wherein the thromboembolism is venous thromboembolism;
[8] a pharmaceutical agent as described in the above [6], wherein the arterial thromboembolism is unstable angina, cardiac infarction, cerebral thrombosis, atherothrombotic cerebral infarction, lacunar infarction, chronic arterial occlusive disease, cardiogenic embolism, peripheral arterial occlusive or embolism attributed to coronary intervention; and
[9] a pharmaceutical agent as described in the above [7], wherein the venous thromboembolism is sinus thrombosis, traveler's thrombosis, pulmonary embolism or deep-vein thrombosis;

More particularly, the present inventors have confirmed previously unknown excellent effects:
1) combination use of cilostazol and [4-[2-(5-amidino-2-hydroxybenzenesulfonylamino)ethyl]-2'-methanesulfonylbiphenyl-3-yloxy]acetic acid shows a significant and excellent effect of synergicly decreasing thrombus formation in the rat thrombus formation inhibition test at dosage of cilostazol, an antiplatelet agent, and [4-[2-(5-amidino-2-hydroxybenzenesulfonylamino)ethyl]-2'-methanesulfonylbiphenyl-3-yloxy]acetic acid, a direct and selective factor Xa inhibitor, either of them does not shown a significant inhibitory effect on thrombus formation by its single application; and
2) while the single use of aspirin, dipyridamole or cilostazol which are antiplatelet agents, or the single use of [4-[2-(5-amidino-2-hydroxybenzenesulfonylamino)-ethyl]-2'-methanesulfonylbiphenyl-3-yloxy]acetic acid both showed no improving effect on hypercoagulable state combination use of them can significantly improve the hypercoagulable state unexpectedly in a confirmatory test of the improvment effect on hypercoagulable state of rabbit blood.

As mentioned above, a pharmaceutical agent comprising an antiplatelet agent in combination with a 5-amidino-2-hydroxybenzenesulfonamide derivative represented by the above general formula (I) shows that 1) synergistically increased effect decreasing the amount of thrombus formation, and 2) a superior improving effect which is not shown by a single application on hypercoagulable state, and therefore, is distinctly useful widely for the patients with thromboembolism.

In addition, a pharmaceutical agent of the present invention makes it possible to carry out the prevention or treatment of thromboembolism sufficiently without increasing the dosage of antiplatelet agent, and to decrease the long-feared risk of bleeding caused by antiplatelet agent as a result of decreasing the dosage of antiplatelet agent. Therefore, a pharmaceutical agent of the present invention is an excellent agent that makes it possible to use an antiplatelet agent, can be used for the patients more safely and comfortably.

As the antiplatelet agent of the present invention, 1) an arachidonic acid metabolism inhibitor (e. g. , aspirin, ozgrel, ethyl icosapentate, dilazep); 2) an ADP receptor inhibitor (e.g., ticlopidine, clopidogrel, prasugrel); 3) a glycoprotein-IIb/IIa antagonist (e.g., abciximab, eptifibatide, tirofiban, lamifiban); 4) a phosphodiesterase inhibitor (e.g., cilostazol, dipyridamole and the like); 5) an adenylcyclase activator (e.g., beraprost sodium, ticlopidine, limaprost, beraprost); 6) a serotonin 5-HT₂ antagonist (e.g., sarpogrelate); 7) a thromboxan A2 receptor antagonist (e.g. , S-18204) and the like can be illustrated, particularly, aspirin, ticlopidine, cilostazol and clopidogrel are preferable.

In the compounds of the above general formula (I) of the present invention, the term "lower alkyl group" means a straight-chained or branched alkyl group having 1 to 6 carbon atoms such as a methyl group, an ethyl group, a propyl group, an isopropyl group, a butyl group, an isobutyl group, a sec-butyl group, a pentyl group, an isopentyl group, a neopentyl group, a 1-methylbutyl group, a 2-methylbutyl group, a hexyl group and the like, and a n-butyl group is most preferable among them. In addition, a hydroxy group is preferable as Z, a compound which has a hydroxy group or a pharmaceutically acceptable salt thereof is a compound indicating good oral absorption.

As the compound represented by the above general formula (I), in particular, n-butyl [4-[2-[2-hydroxy-5-(N-hydroxycarbamimidoyl)benzenesulfonylamino]ethyl-2'-methanesulfonylbiphenyl-3-yloxy]acetate (Compound Ia), [4-[2-[2-hydroxy-5-(N-hydroxycarbamimidoyl)benzenesulfonylamino]ethyl-2'-methanesulfonylbiphenyl-3-yloxy]acetic acid (Compound Ib), n-butyl [4-[2-(5-amidino-2-hydroxybenzenesulfonylamino)ethyl-2'-methanesulfonylbiphenyl-3-yloxy]acetate (Compound Ic) and [4-[2-(5-amidino-2-hydroxybenzenesulfonylamino)ethyl-2'-methanesulfonylbiphenyl-3-yloxy]-acetic acid (Compound Id) are preferable, and particularly, n-butyl [4-[2-[2-hydroxy-5-(N-hydroxycarbamimidoyl)-benzenesulfonylamino]ethyl-2'-methanesulfonylbiphenyl-3-yloxy]acetate (Compound Ia) is most preferable as an oral drug.

As a pharmaceutically acceptable salt of the compound of the above general formula (I) of the present invention, examples of such salts include salts with mineral acids (e.g., hydrochloric acid, hydrobromic acid, hydroiodic acid, nitric acid, sulfuric acid, acetic acid, phosphoric acid and the like), salts with organic acids (e.g., formic acid, acetic acid, methanesulfonic acid, benzenesulfonic acid, p-tosylsulfonic acid, propionic acid, citric acid, succinic acid, tartaric acid, fumaric acid, butyric acid, oxalic acid, malonic acid, maleic acid, lactic acid, malic acid, carbonic acid, glutamic acid, aspartic acid and the like), salts with organic bases (e.g., morpholine, pyrrolidine, piperidine, piperazine, lysine and the like), and salts with inorganic bases such as a sodium salt, a potassium salt and a calcium salt.

In addition, the compounds represented the above general formula (I) also include their hydrates and solvates with pharmaceutically acceptable solvents (e.g., ethanol).

As the preferable combination of the present invention, for example,
1) n-butyl [4-[2-[2-hydroxy-5-(N-hydroxycarbamimidoyl)-benzenesulfonylamino]ethyl]-2'-methanesulfonylbiphenyl-3-yloxy]acetate (Compound Ia) and cilostazol;
2) n-butyl [4-[2-[2-hydroxy-5-(N-hydroxycarbamimidoyl)-benzenesulfonylamino]ethyl]-2'-methanesulfonylbiphenyl-3-yloxy]acetate (Compound Ia) and dipyridamole;
3) n-butyl [4-[2-[2-hydroxy-5-(N-hydroxycarbamimidoyl)-benzenesulfonylamino]ethyl]-2'-methanesulfonyl-biphenyl-3-yloxy]acetate (Compound Ia) and aspirin;
4) n-butyl [4-[2-[2-hydroxy-5-(N-hydroxycarbamimidoyl)-benzenesulfonylamino]ethyl]-2'-methanesulfonylbiphenyl-3-yloxy]acetate (Compound Ia) and ticlopidine; and
5) n-butyl [4-[2-[2-hydroxy-5-(N-hydroxycarbamimidoyl)-benzenesulfonylamino]ethyl]-2'-methanesulfonylbiphenyl-3-yloxy]acetate (Compound Ia) andclopidogrel; can be illustrated, and particularly, a combination of Compound Ia and cilostazol is most preferable as an oral drug.

The prevention of thromboembolism of the present invention includes not only the prevention of the initial occurrence but also the prevention of the reoccurrence. And, the treatment of thromboembolism includes not only the improvement of occurrence condition of the disease but also the prevention of deterioration or progression of occurrence condition of the disease.

As thromboembolism of the present invention, for example,
1) arterial thrombosis: unstable angina, cardiac infarction, cerebral thrombosis, atherothrombotic cerebral infarction, lacunar infarction, chronic arterial occlusive disease, peripheral arterial occlusive or obstruction attributed to coronary intervention (e.g., include coronary balloon angioplasty, coronary stenting, coronary atherectomy and the like);
2) arterial embolism: cardiogenic embolism, lacunar infarction, chronic arterial occlusive disease, arteriosclerotic obliteration, transient ischemic attack, obstruction attributed to coronary intervention (e.g., include coronary balloon angioplasty, coronary stenting, coronary atherectomy and the like), prosthetic vascular replacement, obstruction after prosthetic valve replacement and the like;
3) venous thrombosis: deep-vein thrombosis, sinus thrombosis, postthrombotic syndrome, after thrombosis and the like; and
4) venous embolism: traveler's thrombosis which includes economy class syndrome and the like, pulmonary embolism, deep-vein thrombosis, postthrombotic syndrome after thrombosis and the like can be illustrated. Since a pharmaceutical agent of the present invention is useful for both thrombosis and embolism, a pharmaceutical agent of the present invention can be used for the patients with a wide range of thromboembolism.

The 5-amidino-2-hydroxybenzenesulfonamide derivative represented by the above general formula (I) or a pharmaceutically acceptable salt thereof of the present invention can be prepared, for example , in a manner described in International Publication no.WO02/28827 and the like method.

A pharmaceutical agent of the present invention means an agent comprising an antiplatelet agent in combination with a 5-amidino-2-hydroxybenzenesulfonamide derivative represented by the above general formula (I) or a pharmaceutically acceptable salt thereof, the present invention includes all dosage forms of simultaneous administration as a single preparation, simultaneous administration as separated preparations in way of same or different administration route, and administration at different dosage intervals as separated preparations in way of same or different administration route.

The above "at different dosage intervals" means that the each number of dosage of an antipletele agent and a 5-amidino-2-hydroxybenzenesulfonamide derivative represented by the above general formula (I) or a pharmaceutically acceptable salt thereof can be the same or different, and it is preferable that both agents are administered closely in terms of time in order to achieve the desired preventing or therapeutic effect. And, an order of administration can be decided accordingly depending on the combined drug type. In particular, the pharmaceutical agent of the present invention obtained by combining an platelet agent and a 5-amidino-2-hydroxybenzenesulfonamide derivative represented by the above general formula (I) or a pharmaceutically acceptable salt thereof can be formulated by mixing with pharmaceutically acceptable carriers separately or together in accordance with conventional methods, in the case of formulating separately, can be devoted as a kit of these active ingredients formulated separately. The pharmaceutical agent of the present invention can be used as, for example, 1) a kit consisting of a pharmaceutical composition comprising an antiplatelet agent and a pharmaceutical composition comprising a 5-amidino-2-hydroxybenzenesulfonamide derivative represented by the above general formula (I) or a pharmaceutically acceptable salt thereof; 2) a pharmaceutical composition including an antiplatelet agent and a 5-amidino-2-hydroxybenzenesulfonamide derivative represented by the above general formula (I) or a pharmaceutically acceptable salt thereof; and the like.

An administration route of the pharmaceutical agent of the present invention is not limited in particular, and an antiplatelet agent and a 5-amidino-2-hydroxybenzenesulfonamide derivative represented by the above general formula (I) or a pharmaceutically acceptable salt thereof can be formulated by admixing with a pharmaceutically acceptable carrier individually or together, and can be administered orally or parenterally. As the oral composition, for example, powders, granules, tablets, capsules, dry syrups and the like, as parenteral composition, injections, suppositories, patches and the like can be illustrated.

As the above pharmaceutically acceptable carriers, the various conventional organic or inorganic carriers as drug preparation material can be used, and admixed for preparation, as appropriate excipients, disintegrators, binders, lubricants, diluents, buffers, isotonicities, moistening agents, emulsifiers, dispersing agents, stabilizing agents, solubilizing agents, surface-active agents, dissolving agents, pH regulators, preservatives, antiseptic agents, antioxidizing agents, coloring agents, sweetening agents and the like depending on the forms in accordance with conventional pharmaceutical methods.

For example, powders or granules can be prepared by, if desired, admixing well an active ingredient with appropriate excipients or lubricants

Tablets can be prepared by, if desired, adding to an active ingredient, appropriate excipients, disintegrators, binders, lubricants and the like, and compressing the mixture in accordance with conventional methods. The tablets, furthermore if desired, can be suitably coated to provide film-coated tablets, sugar-coated tablets, enteric-coated tablets and the like.

Capsules can be prepared by, if desired, admixing well an active ingredient with appropriate excipients, lubricants and the like, or preparing granules or fine-powders in accordance with conventional methods, and then filling the preparation in appropriate capsules.

Furthermore, these orally administered compositions can be also derived into immediate- or sustained-release preparation in accordance with the method of treatment.

Injections can be prepared by, if desired, admixing an active ingredient with appropriate solubilizing agents, surface-active agents, dissolving agents, buffers, isotonicities, pH regulators, isotonicities, dispersing agents, antiseptic agents, solubilizing agent and the likeoptionaly.

The dosage of a pharmaceutical agent of the present invention is decided according to the dosage of each active ingredient appropriately depending on the age, body weight and degree of symptoms of each patient, administration time, administration timing, combination of drugs, effect on the treatment and the like, for example, which is approximately within the range of from 150 to 400 mg per day as cilostazol in the case of oral administration, approximately within the range of from 50 to 400 mg per day as dipyridamole in the case of oral administration, approximately within the range of from 150 to 300 mg per day as ticlopidine in the case of oral administration, approximately within the range of from 50 to 320 mg per day as aspirin in the case of oral administration, approximately within the range of from 50 to 300 mg per day as clopidogrel in the case of oral administration. The dosage of a compound represented by the above general formula (I) is used in combination with these antiplatelet agents is approximately within range of from 1 to 1000 mg per day in the case of orally administration and approximately within range of from 0.1 to 500 mg per day in the case of parenteral administration. Since the combination use of an antiplatelet agent and a compound represented by the above general formula (I) can potentiate the efficacy as mentioned above, the dosages of these drugs can be adjusted appropriately, and furthermore, the dosage of the antiplatelet agent can be decreased in accordance with the situation.

### Best Mode for Carrying Out the Invention

The present invention is further illustrated in more detail by way of the following Test Example. However, the present invention is not limited thereto.

### Test Example 1

### The inhibitory effect on the thrombus formation

Rats (Sprague-Dawley rats, male) were divided into four groups of A, B, C and D. 0.5% methylcellulose solution was orally administrated into the rats of groups A and B, and cilostazol (100 mg/kg) was orally administrated into the rats of groups C and D. One hour later, [4-[2-(5-Amidino-2-hydroxybenzenesulfonylamino)ethyl]-2'-methanesulfonylbiphenyl-3-yloxy]aceticacid(compound Id) (3 mg/kg) which dissolved in saline containing 0.05 M sodium hydroxide was subcutaneously administrated into the rats of groups B and D, and saline containing 0.05 M sodium hydroxide subcutaneously administrated into the rats of groups A and C. Simultaneously, the all rats of groups A - D were anesthetized by intraperitoneal injection of 5 mL/kg of 20% urethane solution. After additional one hour, thrombus formation was induced by applying a filter paper (3 x 4 mm) soaked with 50% FeCl₂ solution for 5 minutes to the surface of the abdominal aorta beforehand exposed by the midline incision of the all rats of groups A - D. After removing of the filter paper, each rat was left at rest for additional 25 minutes. Then, the abdominal aorta was ligated at both upper and lower ends of the thrombus induction site. All the thrombus adhering to the inside of the excised abdominal aorta were harvested and dissolved in 1 mL of 0.5 N sodium hydroxide solution as the test solution. The total protein content in the test solution was measured using Coomasie Plus Protein Assay Kit (PIERCE Corp.) as the index of the thrombus formation.

Results were shown in Table 1. The significant inhibitory effects of cilostazol and compound Id alone (group B and group C, respectively) on the thrombus formation was not observed compared with group A as control. On the other hand, in group D (combined use of cilostazol and compound Id), synergistically potentiation of inhibitory effects on the thrombus formation was ascertained compared with group B and C.

Note: "-" mark in Table 1 indicates untreated with test substance, and "*" mark indicates the statistical significant difference with the control group (p < 0.05).

**Table 1**

| Group | Number of animals | Cilostazol | Compound Id | Total protein amount in thrombus (mg) |
|---|---|---|---|---|
| A Control | 4 | - | - | 1.52±0.39 |
| B single application | 4 | - | 3mg/kg | 0.94±0.30 |
| B single application | 3 | 100mg/kg | - | 1.39±0.45 |
| D single application | 3 | 100mg/kg | 3mg/kg | 0.27±0.06* |

### Test Example 2

### The improvement effect on the activated state of blood coagulation

Citrate blood collected from a rabbit (Japanese White, male) was used for the test as the whole blood after maintaining room temperature for 30-60 minutes. 376 µL of the whole blood, 4 µL of a dimethylsulfoxide and 20 µL of a 250 mM calcium chloride solution were poured into a test tube for measurement of activated whole blood clotting time (Hemochron test tube P214, International Technidyne Corporation). After slight stirring of the mixture, an activated whole blood clotting time was measured by a blood clotting meter (Hemochron 801, International Technidyne Corporation) as the control. The independent effect of each antiplatelet drug was examined by similar measuring of the activated whole blood clotting time with use of 2µL of the solution of aspirin, dipyridamole or cilostazol in dimethylsulfoxide which prepared to be set to a final concentration of 1 µM within a test tube and 2 µL of a dimethylsulfoxide, instead of 4 µL of a dimethylsulfoxide in the control. On the other hand, the independent effect of compound Id was examined by similar measuring of the activated whole blood clotting time with use of 2µL of the solution of compound Id in dimethylsulfoxide which prepared to be set to a final concentration of 0.1 µM within a test tube and 2 µL of a dimethylsulfoxide, instead of 4 µL of a dimethylsulfoxide in the control. Furthermore, the combined use effect of each antiplatelet drug and compound Id were examined by similar measuring of the activated whole blood clotting time with combined use of 2µL of the solution of aspirin, dipyridamole or cilostazol in dimethylsulfoxide which prepared to be set to a final concentration of 1 µM within a test tube and 2µL of the solution of compound Id in dimethylsulfoxide which prepared to be set to a final concentration of 0.1 µM in a test tube, instead of 4 µL of a dimethylsulfoxide in the control.

Results were shown in Table 2. Independent use of 1 µM of aspirin, dipyridamole or cilostazol showed a significant prolonged effect on the activated whole blood clotting time to the control (Table 2, B - D). Moreover, the independent use of 0.1 µM of compound Id did not show a significant prolonged effect on the activated whole blood clotting time to the control, either (Table 2, E). On the other hand, only in the combined use of each aspirin, dipyridamole or cilostazol of 1 µM which did not show a significant prolonged effect on the activated whole blood clotting time by independent use, and compound Id of 0.1 µM which did not show a significant prolonged effect on the activated whole blood clotting time by independent use, the significant prolonged effect on the activated whole blood clotting time to the control was ascertained (Table 2, F-H).

Note: "*" mark in Table 2 indicates the statistical significant difference with the control group (p < 0.05)

**Table 2**

| | activated clotting time (seconds, mean ± S.E.) |
|---|---|
| A Control | 218±5.1 |
| B Single Application (Aspirin 1 µM) | 215±16.3 |
| C Single Application (Dipyridamole 1 µM) | 210±10.9 |
| D Single Application (Cilostazol 1 µM) | 205±5.8 |
| E Single Application (Compound Id 0.1 µM) | 228±9.0 |
| F Combination Use (Aspirin 1 µM + Compound Id 0.1 µM) | 253±5.2* |
| G Combination Use (Dipyridamole 1 µM + Compound Id 0.1 µM) | 264±3.5* |
| D Combination Use (Cilostazol 1 µM + Compound Id 0.1 µM) | 245±13.0* |

### Industrial Applicability

The use of a pharmaceutical agent of the present invention combining an antiplatelet agent and a 5-amidino-2-hydroxybenzenesulfonamide derivative represented by the above general formula (I) or a pharmaceutically acceptable salt thereof has a superior feature of synergistically increased effect inhibiting the thrombus formation and improving effect on the hypercoagulable state. Therefore, the pharmaceutical agents are preferable for the prevention or treatment of a wide range of thromboembolism. In addition, the pharmaceutical agents are excellent pharmaceutical agent which can decrease the long-feared risk of bleeding tendency caused by an antiplatelet agent or be used for the patients more safely and comfortably, because they make it possible to carry out the prevention or treatment of thromoembolism sufficiently without increasing the dosage of the antiplatelet agent or decrease the dosage of the antiplatelet agent.

## Claims

1. A pharmaceutical agent comprising an antiplatelet agent in combination with a 5-amidino-2-hydroxybenzenesulfonamide derivative represented by the general formula: 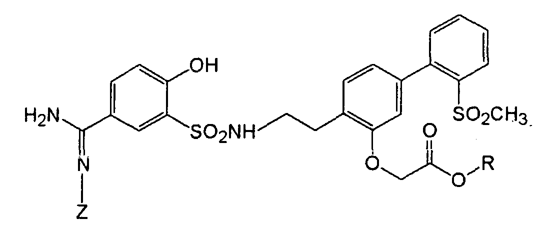 wherein R represents a hydrogen atom or a lower alkyl group; and Z represents a hydrogen atom or a hydroxy group, or a pharmaceutically acceptable salt thereof.

2. A pharmaceutical agent as claimed in claim 1, wherein the antiplatelet agent is a compound selected from the group consisting of aspirin, dipyridamole, cilostazol, ticlopidine and clopidogrel.

3. A pharmaceutical agent as claimed in claim 1, wherein the antiplatelet agent is a compound selected from the group consisting of aspirin, dipyridamole, cilostazol and ticlopidine.

4. A pharmaceutical agent as claimed in claim 1, wherein the 5-amidino-2-hydroxybenzenesulfonamide derivative is a compound selected from the group consisting of n-butyl [4-[2-[2-hydroxy-5-(N-hydroxycarbamimidoyl)benzenesulfonylamino]ethyl]-2'-methanesulfonylbiphenyl-3-yloxy]acetate, [4-[2-[2-hydroxy-5-(N-hydroxycarbamimidoyl)benzenesulfonylamino]ethyl]-2'-methanesulfonylbiphenyl-3-yloxy]acetic acid, n-butyl [4-[2-(5-amidino-2-hydroxybenzenesulfonylamino)-ethyl]-2'-methanesulfonylbiphenyl-3-yloxy]acetate and [4-[2-(5-amidino-2-hydroxybenzenesulfonylamino)-ethyl]-2'-methanesulfonylbiphenyl-3-yloxy]acetic acid.

5. A pharmaceutical agent as claimed in any one of claims 1 to 4, which is an agent for the prevention or treatment of thromboembolism.

6. A pharmaceutical agent as claimed in calim 5, wherein the thromboembolism is arterial thromboembolism.

7. A pharmaceutical agent as claimed in claim 5, wherein the thromboembolism is venous thromboembolism.

8. A pharmaceutical agent as claimed in claim 6, wherein the arterial thromboembolism is unstable angina, cardiac infarction, cerebral thrombosis, atherothrombotic cerebral infarction, lacunar infarction, chronic arterial occlusive disease, cardiogenic embolism, peripheral arterial occlusive or embolism attributed to coronary intervention.

9. A pharmaceutical agent as claimed in claim 7, wherein the venous thromboembolism is sinus thrombosis, traveler's thrombosis, pulmonary embolism or deep-vein thrombosis.
